# EUROPÄISCHE PATENTSCHRIFT

(11) **EP 0 765 144 B1**
(45) Veröffentlichungstag und Bekanntmachung des Hinweises auf die Patenterteilung: **26.08.1998**
(21) Anmeldenummer: 95923292.7
(22) Anmeldetag: 10.06.1995
(51) Int. Cl.: A61F 2/01

(54) **VENA-CAVA THROMBEN-FILTER**
VENA CAVA THROMBUS FILTER
FILTRE A THROMBUS POUR VEINE CAVE

(30) Priorität: 11.06.1994 DE 9409484 U
(43) Veröffentlichungstag der Anmeldung: 02.04.1997
(73) Patentinhaber: Naderlinger, Eduard, 50127 Bergheim (DE)
(72) Erfinder: Naderlinger, Eduard, 50127 Bergheim (DE)
(74) Vertreter: Mey, Klaus-Peter, Dr.-Ing. Dipl.-Wirtsch.-Ing.
(86) Internationale Anmeldenummer: EP9502244
(87) Internationale Veröffentlichungsnummer: WO9534254

(56) Entgegenhaltungen:
- EP-A- 0 117 940
- EP-A- 0 165 713
- EP-A- 0 472 334
- EP-A- 0 521 222
- EP-A- 0 598 635
- FR-A- 2 643 250
- FR-A- 2 652 267
- BIEDERBICK,KARLHEINZ 'Kunststoffe', 1977, VOGEL, WUERZBURG

## Beschreibung

Die Erfindung betrifft ein Vena-cava Thromben-Filter mit einem zum Einführen in eine Vene ausgebildeten Katheter, der ein Röhrchen aus flexiblem Kunststoff mit einem darin axial verschieblich gelagerten Führungs- und Haltedraht besitzt, dessen Einführungsende ein Filterelement aufweist, das aus dünnen, innerhalb einer Vene elastisch aufspreizbaren Drähten besteht, die sich in vorgeschobener Position außerhalb des Katheter-Einführungsendes zur Form eines flüssigkeitsdurchlässigen Korbes aufspreizen.

Die Indikation zum Einsatz eines Vena-cava-Filters ergibt sich zwingend beispielsweise bei Thrombolysen zur temporären Prophylaxe von Lungenembolien durch sich lösende Thromben bzw. Teile solcher Thromben.

Bekannte Vorrichtungen, um derartige Thromben im Blutkreislauf aufzufangen und/oder zu entfernen, sind zumeist aus Kunststoff und können bei Belastung abknicken, wodurch ihre Funktion beeinträchtigt wird. Vielfach sind die bekannten Vena-Cava Thromben-Filter auch kompliziert in Aufbau und Handhabung.

Aus der EP-A-0 472 334 ist eine Vorrichtung zum Filtern von Blut in Blutgefäßen eines Patienten bekannt. Sie umfaßt einen Katheter mit einem darin axial verschiebbar gelagerten Führungs- und Haltedraht, an dessen distalem Ende ein aufspreizbares Filterelement angeordnet ist, das aus mehreren in Form eines Korbes aufspreizbaren Drähten besteht, die je nach axialer Verschiebung des Führungsdrahtes innerhalb eines Blutgefäßes zum Filter aufspannbar oder zusammenziehbar ist.

Die EP-A-0 117 940 beschreibt eine ähnliche Vorrichtung mit einem Thromben-Filter am distalen Ende eines in eine Vene einführbaren Katheters. Bei dieser Vorrichtung besteht das Filterelement aus schlingenförmig kräuselbaren Memory-Drähten, die mit Hilfe des Führungsdrahtes durch Strecken beim Einführen in die Vene zusammenlegbar oder durch Gegenbewegung des Führungsdrahtes zur Schlingenform in der Vene ausbreitbar sind. Mit Hilfe einer Abzweigung der Armatur am Führungsende kann durch den Katheter eine Injektionsflüssigkeit in die Vene injiziert werden.

Der Erfindung liegt die Aufgabe zugrunde, einen temporären Vena-cava Thromben-Filter mit einem zum Einführen in eine Vene ausgebildeten Katheter zu verbessern bzw. zur Verfügung zu stellen, mit welchem es gelingt, bei einer Thrombolyse, Thrombektomie oder Risikoschwangerschaft sich ablösende Thromben oder Teile davon aus dem Blutkreislauf auszufiltern, um der Gefahr einer Lungenembolie sicher vorzubeugen. Der Filter soll dabei so beschaffen sein, daß mit ihm wahlweise ein Zugang über alle bekannten Zugänge, z. B. Armvenen (v.-basilica, -brachialis, v.-jugularis, -subclavia, -femoralis) möglich ist. Demnach soll der Katheter wie ein Angiografiekatheter sehr dünn und flexibel sein und eine Liegedauer während der gesamten Lysezeit möglich machen.

Die Lösung der gestellten Aufgabe gelingt bei einem Vena-cava Thromben-Filter mit einem zum Einführen in eine Vene ausgebildeten Katheter mit der Erfindung durch dessen Ausgestaltung entsprechend den kennzeichnenden Merkmalen von Anspruch 1.

Mit großem Vorteil findet der Vena-cava Thromben-Filter im geschlossenem Zustand mit durch Einzug in das distale Ende des Katheter-Röhrchens zusammengefaltet elastischen Drähten Zugang in alle wichtigen Venen und Venenstränge, wobei nach erfolgter und im Röntgenbild überprüfter Positionierung durch Betätigen des Führungs- und Haltedrahtes das eigentliche Filterelement aus dem Ende des Katheterröhrchens herausgeschoben und in die aufgespreizte Position versetzt wird. Dabei entfalten dann die elastisch aufspreizbaren Drähte das korbförmige Filterelement, welches geeignet ist, im Blutkreislauf flotierende Thromben oder Teile davon aufzuhalten bzw. einzufangen.

Weitere zweckmäßige Ausgestaltungen des Vena-cava Thromben-Filters sind entsprechend den Unteransprüchen vorgesehen.

Die Erfindung wird in schematischen Zeichnungen in einer bevorzugten Ausführungsform gezeigt, wobei aus den Zeichnungen weitere vorteilhafte Einzelheiten der Erfindung entnehmbar sind.

Es zeigen:
- Fig. 1: in Seitenansicht einen Vena-cava Thromben-Filter mit ausgefahrenem, elastisch aufgespreiztem Filterelement,
- Fig. 2: in Frontansicht und teilweise im Schnitt den Thromben-Filter mit ausgefahrenem und aufgepreiztem Filterelement,
- Fig. 3: eine Seitenansicht des Thromben-Filters mit in den Katheter eingefahrenem, zum Einführen in eine Vene vorgesehenem Zustand.

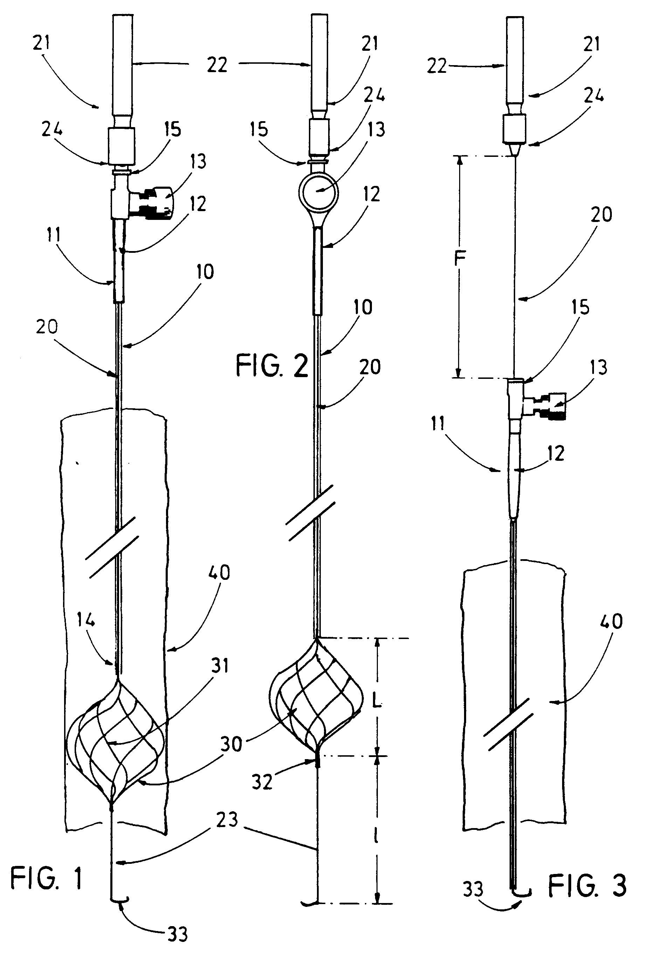

Der in Figur 1 gezeigte Vena-cava Thromben-Filter weist ein nach Einführung in eine Vene (40) aus dem Einführungsende (14) ausgefahrenes und nach Austritt aus dem Einführungsende (14) durch die Elastizität der dünnen, aufspreizbaren Drähte (31) in Form eines Korbes aufgespreiztes Filterelement (30) auf.

Vor Verwendung des erfindungsgemäßen Vena-cava-Filters wird zunächst eine Seldingernadel in eine Armvene eingeführt, durch die ein kurzer Führungsdraht geschoben wird, wonach die Nadel über den Führungsdraht hinweg wieder entfernt wird. Danach wird eine handelsübliche 5 F-Schleuse in die Vene implantiert und der Führungsdraht mit Dilator herausgenommen. Durch die Schleuse wird nunmehr das Führungsende (14) des Katheters (10) bei eingezogenem Filterelement (30) in die Vene eingeschoben.

Wie die Zusammenschau der Figuren 1 bis 3 zeigt, ist der Katheter (10) ein Röhrchen aus flexiblem Kunststoff mit einem darin axial verschieblich gelagerten Führungs- und Haltedraht (20). Am Handhabungsende (21) des Führungs- und Haltedrahtes (20) ist ein Handgriff (22) vorgesehen, mit dem das Filterelement (30) einziehbar oder aufspannbar ist. Durch Herausziehen bzw. Hineindrücken des Handgriffs (22) wird der Katheter (10) bewegt und das Filterelement (30) geschlossen oder geöffnet. So zeigen beispielsweise die Figuren 1 und 2 bei eingeschobenem Führungsdraht (20) das aus dem Katheterende (14) ausgefahrene und durch Elastizität der in Helixform aufspannbaren Drähte (31) in Form eines Korbes aufgespreizte Filterelement (30), während Figur 3 den eingezogenen Zustand zeigt, der sich bei herausgezogenem Handgriff (22) des Katheters (10) einstellt. Das Filterelement (30) hat im aufgespreizten Zustand die Funktion eines flüssigkeitsdurchlässigen Siebes in Form eines Korbes (30). Es besteht aus mehreren, bevorzugt aus acht bauchig in Helixform aufspannbaren Drähten (31) von implantierbarer Memorydrahtqualität mit etwa 0,08 mm Durchmesser.

Eine zweckmäßige Ausbildung sieht vor, daß am Ende des den Filter bildenden Drahtkorbes (30) eine Hülse (32) angeordnet ist, an der ein mit Teflon bzw. Polytetrafluoräthylen (Teflon = eingetragenes Warenzeichen) überzogener Führungsdraht (23) mit einer J-Führungsdrahtspitze (33) befestigt ist.

Eine weitere zweckmäßige Ausgestaltung sieht vor, daß der Katheter (10) an seinem Handhabungsende (21) über der metallischen Führungshülse der Armatur (13) und dem darauf aufgezogenen Katheterröhrchen (10) mit einer flexiblen Gummi- oder Kunststoffhülse (12) abgedeckt ist. Die Armatur (13) ist für ein Spülfluid verschließbar ausgebildet. Weiterhin weist die Armatur (13) Mittel (15) auf, an welchen der Handgriff (22) des Führungs- und Haltedrahtes (20) mit einem Bajonettverschluß (24) festlegbar ist.

Um auch bei vergrößerten Venendurchmessern das Filterelement (30) sicher einsetzen zu können, weist dieses einen Durchmesser von ca. 35 mm und eine Länge von ca. 55 mm auf. Es ist jedoch ersichtlich, daß das Filterelement (30) wegen der außerordentlichen Elastizität seiner extrem dünnen Drähte (31) sich an jeden Venendurchmesser problemlos anpaßt und auch in sehr dünnen Venen einsetzbar ist. Besonders vorteilhaft kann es mittels einer handelsüblichen 5 F-Schleuse implantiert werden.

Eine sichere Betätigung durch Herausziehen und Hineindrücken des Führungs- und Haltedrahtes (20) wird dadurch erreicht, daß der Draht (20) relativ zum umgebenden Katheterröhrchen (10) mit axialem Freigang (F) gemäß Fig. 3 ausgebildet ist und dieser zumindest der Länge (L) des aufgespreizten Filters (30) zuzüglich der Länge (l) der endständigen Hülse (32) einschließlich der ebenfalls mit Polytetrafluoräthylen überzogenen J-Führungsdrahtspitze (33) entspricht.

Der erfindungsgemäß temporär anwendbare Vena-cava-Filter erfüllt mit der gezeigten Ausbildung alle Anforderungen für einen optimalen Thrombolyseschutz. Er ermöglicht wahlweisen Zugang über alle bekannten Zugänge wie z. B. Armvenen, ist sehr dünn, beispielsweise mit einem Außendurchmesser des Katheters von 1,5 bis 2 mm und extrem flexibel. Er ist für alle cava-Größen geeignet und kann auch in die v-lliaca gelegt werden. Weiterhin ermöglicht die Bauart und die durch den Bajonettverschluß (24) mögliche Feststellbarkeit eine längere Liegedauer während einer Lysezeit, wobei auch noch die integrierte J-Führungsdrahtspitze ein sehr einfaches Legen ermöglicht.

Insgesamt ergibt die erfindungsgemäße Ausbildung des Vena-Cava Thromben-Filters mit dem zum Einführen in eine Vene ausgebildeten Katheter eine optimale Lösung der eingangs gestellten Aufgabe.

## Patentansprüche

1. Vena-Cava Thromben-Filter mit einem zum Einführen in eine Vene (40) ausgebildeten Katheter (10), der ein Röhrchen aus flexiblem Kunststoff mit einem darin axial verschieblich gelagerten Führungs- und Haltedraht (20) besitzt, dessen distales Ende (23) ein Filterelement (30) aufweist, das aus innerhalb einer Vene elastisch aufspreizbaren Drähten (31) besteht, die sich in vorgeschobener Position außerhalb des Katheter-Einführungsendes (14) zur Form eines flüssigkeitsdurchlässigen Korbes (30) aufspreizen, **dadurch gekennzeichnet**, daß der Katheter (10) am Handhabungsende (21) des Führungs- und Haltedrahtes (20) mit einem Handgriff (22) versehen ist, wobei über einer metallischen Führungshülse an einer für ein Spülfluid verschließbaren Armatur (13) und dem darauf aufgezogenen Katheterröhrchen (10) eine flexible Gummi- oder Kunststoffhülse (12) angeordnet ist und die Armatur (13) Mittel (15) aufweist, an welchen der Handgriff (22) des Führungs- und Haltedrahtes (20) mit einem Bajonettverschluß festlegbar ist.

2. Vena-Cava Thromben-Filter nach Anspruch 1, **dadurch gekennzeichnet**, daß der Führungs- und Haltedraht (20) relativ zum umgebenden Katheterröhrchen (10) mit axialem Freigang (F) ausgebildet ist, und daß am distalen Ende des das Filterelement bildenden Drahtkorbes (30) eine Hülse (32) aus Metall und an dieser ein mit Polytetrafluoräthylen überzogener Führungsdraht (23) mit einer ebenfalls mit Polytetrafluoräthylen überzogenen J-Führungsdrahtspitze (33) angeordnet ist und der axiale Freigang (F) zumindest der Länge (L) des aufgespreizten Filterelementes (30) zuzüglich der Länge (l) der endständigen Hülse (32) einschließlich der J-Führungsdrahtspitze (33) entspricht.

3. Vena-Cava Thromben-Filter nach Anspruch 1 oder 2, **dadurch gekennzeichnet**, daß das zur Form eines Korbes (30) aufspreizbare Filterelement einen Durchmesser von ca. 35 mm und eine Länge von ca. 55 mm aufweist.

4. Vena-Cava Thromben-Filter nach Anspruch 3, **dadurch gekennzeichnet**, daß das Filterelement (30) aus mehreren, bevorzugt aus acht bauchig in Helixform aufspannbaren Drähten (31) von implantierbarer Memorydrahtqualität mit 0,08 mm Durchmesser besteht.

## Claims

1. Vena cava clot filter with a catheter, which is designed for insertion into a vein (40) and comprises a narrow tube made of flexible plastic material, in which a guiding and holding wire (20) is mounted in an axially displaceable manner, the distal end (23) of which has a filter element (30) comprising wires (31) elastically spreadable inside a vein, which wires in an advanced position outside of the catheter insertion end (14) spread into the shape of a liquid-permeable basket (30), characterized in that the catheter (10) at the operating end (21) of the guiding and holding wire (20) is provided with a handle (22), a flexible rubber or plastic sleeve (12) being disposed over a metal guide sleeve of a fitting (13) closable for an irrigating fluid and over the catheter tube (10) mounted thereon, and the fitting (13) having means (15), to which the handle (22) of the guiding and holding wire (20) is fastenable by means of a bayonet catch.

2. Vena cava clot filter according to claim 1, characterized in that the guiding and holding wire (20) is designed with axial freedom of movement (F) relative to the surrounding catheter tube (10), and that disposed at the distal end of the wire basket (30) forming the filter element is a sleeve (32) made of metal and disposed on said sleeve is a guiding wire (23) coated with polytetrafluoroethylene and having a J-shaped guiding wire tip (33) likewise coated with polytetrafluoroethylene and the axial freedom of movement (F) corresponds at least to the length (L) of the spread filter element (30) plus the length (l) of the end sleeve (32) including the J-shaped guiding wire tip (33).

3. Vena cava clot filter according to claim 1 or 2, characterized in that the filter element spreadable into the shape of a basket (30) has a diameter of around 35 mm and a length of around 55 mm.

4. Vena cava clot filter according to claim 3, characterized in that the filter element (30) comprises a plurality of, preferably eight, wires (31) of implantable memory wire quality having a diameter of 0.08 mm, which are spreadable convexly into a helical shape.

## Revendications

1. Filtre à thrombus pour veine cave avec un cathéter (10) réalisé pour l'introduction dans une veine (40) qui comprend un petit tube en matière plastique flexible avec un fil de guidage et de support (20) monté coulissable axialement dans celui-ci, dont l'extrémité distale (23) a un élément de filtre (30), qui est composé de fils (31) pouvant s'écarter élastiquement à l'intérieur d'une veine fils qui s'écartent en position avancée à l'extérieur des l'extrémité d'introduction du cathéter (14) en forme d'un panier perméable aux liquides,
caractérisé en ce que
le cathéter (10) est muni à l'extrémité servant au maniement (21) du fil de guidage et support (20) d'une poignée (22), un manchon de caoutchouc ou de matière plastique (12) flexible étant disposé par l'intermédiaire d'un manchon de guidage métallique contre une armature (13) verrouillable pour un fluide de lavage, et sur le petit tube de cathéter (10) enfilé vers le haut et l'armature (13) comporte des moyens (15), sur lesquels la poignée (22) du fil de guidage et de support (20) peut être fixée avec une fermeture à baïonnette.

2. Filtre à thrombus pour veine cave selon la revendication 1,
caractérisé en ce que
- le fil de guidage et de support (20) est réalisé relativement au petit tube de cathéter (10) l'entourant avec une zone libre axiale (F) et
- à l'extrémité distale du panier de fil (30) formant l'élément de filtre est disposé un manchon (32) en métal et dans celui-ci un fil de guidage (23) revêtu de polytétrafluoréthylène avec une pointe du fil (33) de guidage J revêtue également de polytétrafluoréthylène et la zone livre axiale (F) correspond au moins à la longueur (L) de l'élément de filtre (30) écarté plus la longueur (l) du manchon (32) situé à l'extrémité y compris la pointe de fil (33) de guidage en J.

3. Filtre à thrombus pour veine cave selon la revendication 1 ou 2,
caractérisé en ce que
l'élément de filtre pouvant s'écarter en forme de panier (30) comprend un diamètre d'environ 35 mm et une longueur d'environ 55 mm.

4. Filtre à thrombus pour veine cave selon la revendication 3,
caractérisé en ce que
l'élément de filtre (30) est composé de plusieurs, de préférence de huit fils (31) pouvant s'écarter de manière bombée sous forme d'hélice, de qualité de fil memory pouvant être implantée, ayant 0,08 mm de diamètre.
